# EUROPEAN PATENT SPECIFICATION

(11) **EP 1 723 938 B1**
(45) Date of publication and mention of the grant of the patent: **07.05.2008**
(21) Application number: 06016108.0
(22) Date of filing: 18.07.2003
(51) Int. Cl.: A61F 2/92

(54) **Stent coating holder**
Haltevorrichtung zur Beschichtung eines Stents
Support pour le revêtement d'une endoprothèse vasculaire

(30) Priority: 19.07.2002 US 198094
(43) Date of publication of application: 22.11.2006
(62) Divisional of application: 03748951.5
(73) Proprietor: Boston Scientific Limited, St. Michael, Barbados, West Indies (BB)
(72) Inventor: Epstein, Samuel, Watertown, MA 02169 (US); Hansen, Henrik, 3751 Astermariem (DK); Coyle, Simon, Galway (IE); Grenham, Niall, Galway (IE); Hayes, Micheal, Galway (IE); Serrano, Gabriel Sobrino, Galway (IE)
(74) Representative: Altenburg, Udo

(56) References cited:
- EP-A- 0 221 001
- EP-A- 0 897 701
- DE-A1- 10 037 337
- US-A- 5 922 393

## Description

### Field Of The Invention

The present invention generally regards the holding of stents during manufacture to enable the application of therapeutic and/or protective coatings. More specifically, the present invention provides stent holders that securely retain stents during the application of a coating while minimizing compressive and tensile forces applied to the stents and disruptions to the coating due to holder blockage of coating deposition.

### Background

Medical implants are used for innumerable medical purposes, including the reinforcement of recently re-enlarged lumens, the replacement of ruptured vessels, and thetreatment of disease such as vascular disease by local pharmacotherapy, i. e., delivering therapeutic drug doses to target tissues while minimizing systemic side effects. Such localized delivery of therapeutic agents has been proposed or achieved using medical implants which both support a lumen within a patient's body and place appropriate coatings containing absorbable therapeutic agents at the implant location.

The term "therapeutic agent" as used herein includes one or more "therapeutic agents" or "drugs". The terms "therapeutic agents" and "drugs" are used interchangeably herein and include pharmaceutically active compounds, nucleic acids with and without carrier vectors such as lipids, compacting agents (such as his-tones), virus (such as adenovirus, andenoassociated virus, retrovirus, lentivirus and a-virus), polymers, hyaluronic acid, proteins, cells and the like, with or without targeting sequences.

Specific examples of therapeutic agents used in conjunction with the present invention include, for example, pharmaceutically active compounds, proteins, cells,oligonucleotides, ribozymes, anti-senseoligonucleotides, DNA compacting agents,gene/vector systems (i. e. , any vehicle that allows for the uptake and expression of nucleic acids), nucleic acids (including, for example, recombinant nucleic acids; naked DNA, cDNA, RNA; genomic DNA, cDNA or RNA in a non-infectious vector or in a viral vector and which further may have attached peptide targeting sequences; antisense nucleic acid (RNA or DNA); and DNA chimeras which include gene sequences and encoding for ferry proteins such as membrane translocating sequences ("MTS") and herpes simplex virus-1 ("VP22"), and viral, liposomes and cationic and anionic polymers and neutral polymers that are selected from a number of types depending on the desired application. Non-limiting examples of virus vectors or vectors derived from viral sources include adenoviral vectors, herpes simplex vectors, papilloma vectors, adeno-associated vectors, retroviral vectors, and the like. Non-limiting examples of biologically active solutes include anti-thrombogenic agents such as heparin, heparin derivatives, urokinase, and PPACK (dextrophenylalanine proline arginine chloromethylketone); antioxidants such as probucol and retinoicacid; angiogenic and anti-angiogenic agents and factors; agents blocking smooth muscle cell proliferation such asrapamycin, angiopeptin, and monoclonal antibodies capable of blocking smooth muscle cell proliferation; anti-inflammatory agents such as dexamethasone, prednisolone, corticosterone, budesonide, estrogen, sulfasalazine, acetyl salicylic acid, and me-salamine; calcium entry blockers such asverapamil, diltiazem and nifedipine; antineoplastic/antiproliferative/anti-mitotic agents such aspaclitaxel, 5-fluorouracil, methotrexate,doxorubicin, daunorubicin, cyclosporine, cisplatin, vinblastine, vincristine, epothilones, endostatin, angiostatin and thymidine kinase inhibitors; antimicrobials such as triclosan, cephalosporins, aminoglycosides, and nitrofurantoin; anesthetic agents such as lidocaine, bupivacaine, and ropivacaine; nitric oxide (NO) donors such as linsidomine, molsidomine, L-arginine, NO-protein adducts, NO-carbohydrate adducts, polymeric or oligomeric NO adducts; anti-coagulants such as D-Phe-Pro-Arg chloromethyl ketone, an RGD peptide-containing compound, heparin, antithrombin compounds, platelet receptor antagonists, anti-thrombin antibodies, anti-platelet receptor antibodies, enoxaparin, hirudin, warafin sodium, Dicumarol, aspirin, prostaglandin inhibitors, platelet inhibitors and tick antiplatelet factors; vascular cell growth promoters such as growth factors, growth factor receptor antagonists, transcriptional activators, and translational promoters; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; agents which interfere with endogeneus vasoactive mechanisms; survival genes which protect against cell death, such as anti-apoptoticBcl-2 family factors and Akt kinase; and combinations thereof. Cells can be of human origin (autologous or allogenic) or from an animal source(xenogeneic), genetically engineered if desired to deliver proteins of interest at the insertion site. Any modifications are routinely made by one skilled in the art.

Polynucleotide sequences useful in practice of the invention include DNA or RNA sequences having a therapeutic effect after being taken up by a cell. Examples of therapeutic polynucleotides include anti-sense DNA and RNA; DNA coding for an anti-sense RNA; or DNA coding for tRNA or rRNA to replace defective or deficient endogenous molecules. The polynucleotides can also code for therapeutic proteins or polypeptides. A polypeptide is understood to be any translation product of a polynucleotide regardless of size, and whether glycosylated or not. Therapeutic proteins and polypeptides include as a primary example, those proteins or polypeptides that can compensate for defective or deficient species in an animal, or those that act through toxic effects to limit or remove harmful cells from the body. In addition, the polypeptides or proteins that can be injected, or whose DNA can be incorporated, include without limitation, angiogenic factors and other molecules competent to induce angiogenesis, including acidic and basic fibroblast growth factors, vascular endothelial growth factor,hif-1, epidermal growth factor, transforming growth factora and platelet-derived endothelial growth factor, platelet-derived growth factor, tumor necrosis factoroc, hepatocyte growth factor and insulin like growth factor; growth factors; cell cycle inhibitors including CDK inhibitors; anti-restenosis agents, including p 15, p 16, p 18, p 19, p21, p27, p53, p57, Rb, nFkB and E2F decoys, thymidine kinase ("TK") and combinations thereof and other agents useful for interfering with cell proliferation, including agents for treating malignancies; and combinations thereof. Still other useful factors, which can be provided as polypeptides or as DNA encoding these polypeptides, include monocyte chemoattractant protein ("MCP-1"), and the family of bone morphogenic proteins ("BMP's"). The known proteins include BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively or, in addition, molecules capable of inducing an upstream or downstream effect of a BMP can be provided. Such molecules include any of the "hedgehog" proteins, or the DNA's encoding them.

The delivery of expandable stents is a specific example of a medical procedure that involves the deployment of coated implants. Expandable stents are tube-like medical devices, typically made from stainless steel, Tantalum, Platinum or Nitinol alloys, designed to be placed within the inner walls of a lumen within the body of a patient. These stents are typically maneuvered to a desired location within a lumen of the patient's body and then expanded to provide internal support for the lumen. The stents may be self-expanding or, alternatively, may require external forces to expand them, such as by inflating a balloon attached to the distal end of the stent delivery catheter.

Because of the direct contact of the stent with the inner walls of the lumen, stents have been coated with various compounds and therapeutic agents to enhance their effectiveness. These coatings may, among other things, be designed to facilitate the acceptance of the stent into its applied surroundings. Such coatings may also be designed to facilitate the delivery of one of the foregoing therapeutic agents to the target site for treating, preventing, or otherwise affecting the course of a disease or tissue or organ dysfunction.

Where the stent has been coated, care must be taken during its manufacture and delivery within the patient to ensure the coating is evenly applied and firmly adherent to the stent, and further that the coating is not damaged or completely removed from the implant during the deployment process. When the amount of coating is depleted the implant's effectiveness may be compromised and additional risks may be inured into the procedure.

For example, when the coating of the implant includes a therapeutic, if some of the coating were removed during deployment, the therapeutic may no longer be able to be administered to the target site in a uniform and homogenous manner. Thus, some areas of the target site may receive high quantities of therapeutic while others may receive low quantities of therapeutic. Similarly, if the therapeutic is ripped from the implant it can reduce or slow down the blood flowing past it, thereby, increasing the threat of thrombosis or, if it becomes dislodged, the risk of embolisms. In certain circumstances, the removal and reinsertion of the stent through a second medical procedure may be required where the coatings have been damaged or are defective.

The mechanical process of applying a coating onto a stent may be accomplished in a variety of ways, including, for example, the spraying of the coating substance onto the stent and so-called spin-dipping, i. e., dipping a spinning stent into a coating solution to achieve the desired coating. Common to these processes is the need to securely hold the stent in a desired orientation during the process of coating application to ensure an intact, robust coating of the desired thickness is formed on the stent.

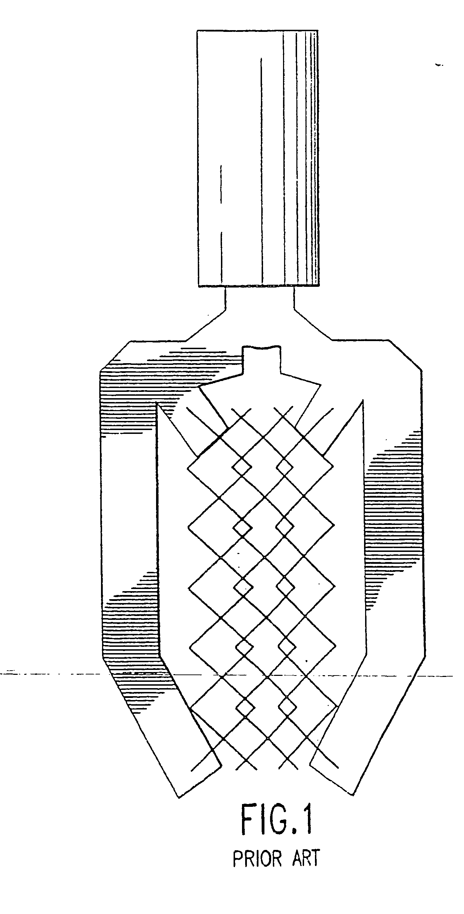

If the stent is held too loosely, it may either shift during the coating process or it may become prematurely separated from the holder, resulting in an inconsistent or damaged coating. For example, Fig.1 illustrates a prior art spin-dipping stent holder 1 with a grappling clip bent in a manner that allows the stent to be maneuvered into the grappling clip's grasp. Difficulties with properly aligning the stent on this device, high centripetal forces generated during spinning, and low retention forces on the stent can result in premature separation of the stent from the holder. Further, this device is not is suitable for universal use across a range of stent sizes, as it must be custom built for each specific stent size.

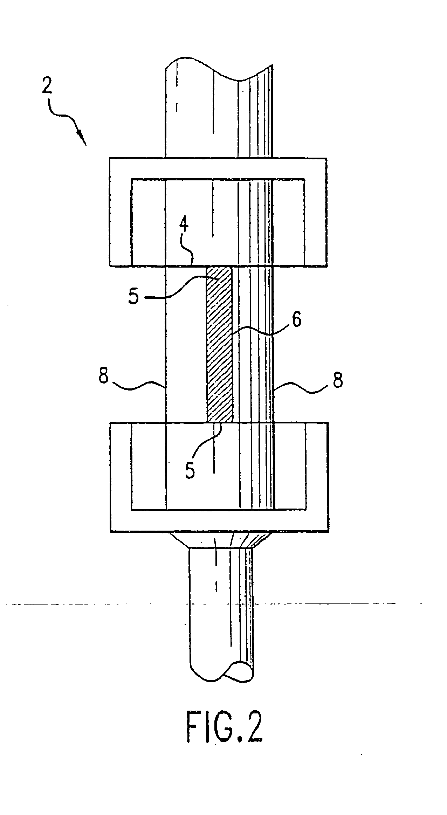

On the other hand, if the stent is held with too great a compressive or tensile force, it and/or its supporting structure may buckle, collapse or prematurely expand. For example, Fig. 2 illustrates another prior art stent holder 2 in which two tensioned cross wires 4 compress the stent ends 5 to hold stent 6 in place during coating spray application. Due to the need to generate substantial compression force on the stent to hold it in place, there occur problems such as mid-section bulging or buckling of longer stents, stent misalignment and accelerated wear and slippage of the wires and bushings used to secure the wires to the holder (not shown). In addition, due to the relatively large tensile loading, the support struts8 must be made sufficiently large to avoid failure, which in turn can result in inadequate coating formation on the stent due to spray "shadowing", i.e., incomplete coating spray application onto the stent due to structural elements blocking the spray. An additional disadvantage of this type of stent holder is its relatively high expense, given its complexity and the need to use high strength materials.

US 5,922,393, which represents the closest prior art, discloses a coated microporous stent and method of coating are disclosed. The stent is arranged on a mandrel and then dipped in a container of coating material. Thereafter, the stent is programmed in the desired manner and is subsequently physically compressed and kept inside a sheath.

Thus, there is a need for a relatively inexpensive, robust stent holder which can positively locate and retain a stent during stent coating processes such as spin-dipping and spray coating, while not interfering with the application of the coating.

### Summary of The Invention

The object of the present invention is to provide a stent holder for holding a stent during application of a stent coating which avoids stent movements during coating, and which minimized holder shadowing.

This object is solved by a stent holder according to claim 1. The dependent claims depict advantageous embodiments of the present invention.

According to the present invention, a frame is equipped with two opposing "V"-shaped wires, between which a stent may be placed. The stent is preferably mounted between the tips of the "V"-shaped wires such that, like the previous embodiments, the stent is firmly held at two interior radial contact locations at each end of the stent to preclude movement during the stent coating process. The stent may be easily inserted into this holder by flexing the holder frame so that the "V"-shaped wire tips separate sufficiently to permit placement of the stent between the tips, and then releasing the frame to permit the wire tips to apply a light compressive force on the stent ends. As with the prior embodiments, this embodiment also avoids coating spray blockage, and has the further advantage of simple and efficient stent loading and virtually unlimited reusability.

### Brief Description of The Drawings

Fig. 1 is an illustration of a prior art spin-dipping stent coating holder.
Fig. 2 is an illustration of a prior art spray deposition stent coating holder.
Fig. 3 is an oblique view of a first example of a stent coating holder.
Fig. 4 is a side view of the wire of the first example of a stent coating holder.
Fig. 5 is a detail side view of the arrangement of the wire loop sides within a stent held in the first example of a stent coating holder.
Fig. 6 is a side view of a second example of a stent coating holder.
Fig. 7 is a side view of the stent coating holder of Fig. 6 following rotation through 90 degrees about the stent coating holder's longitudinal axis.
Fig. 8 is a side view of a portion of the center rod of the second example of a stent coating holder.
Fig 9 is a cross-section view of a third example of a stent holder with a stent mounting thereon.
Fig. 10 is a schematic view of the third example of a stent holder affixed to a carrier of a tape and reel production process apparatus.
Fig. 11 is a side view of an embodiment of a stent coating holder in accordance with the present invention.

The stent holders shown in Figs. 1 to 10 do not form a part of the invention as claimed.

### Detailed Description

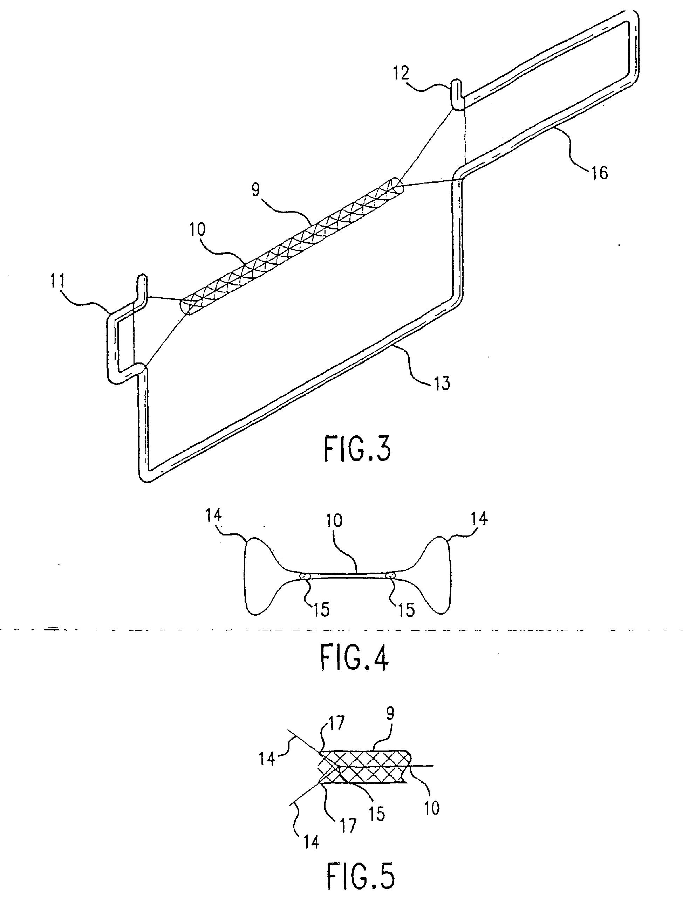

Fig.3 shows a first example of a stent coating holder. As shown in Fig. 3, stent 9 is suspended on stent holding wire 10. Wire 10 passes through the center of stent 9 and is secured to two loop holders 11 and 12 at opposing ends of stent holding frame 13.

Stent holding wire 10 in this example is formed as shown in Fig. 4. Each end of wire 10 is formed into a loop 14, with the end of each loop secured to wire 10 at a position 15 along the wire that results in a loop of a specific desired size. Stent holding wire 10 is formed from a material possessing sufficient tensile strength to support the stent during stent coating, and possessing sufficient elasticity to permit stent holding wire 10 to be readily formed into loops at its opposing ends of wire 10 and to permit a loop 14 to be collapsed in order to be inserted through the center of stent 9 when preparing to place the stent on the stent coating holder. In this example, stent holding wire 10 is preferably formed from stainless steel wire with a diameter of 50.8 µm [0.002 inches], and the loops 14 are secured to wire 10 by welding.

Stent holding frame 13 is formed by bending a rod into the desired shape. The rod material should have sufficient resistance to bending such that frame 13 will be able to maintain adequate axial tension on stent holding wire 10 to securely retain the stent thereon, yet not be so stiff as to prevent the securing of the wire loops 14 over loop holders 11 and 12. The rod material must also be sufficiently resistant to bending to ensure that loop holders 11 and 12 will be able to keep their respective wire loops 14 adequately spread apart.

In this example, the holder is preferably constructed from a 1.0 mm diameter stainless steel rod press-formed into the desired shape. As shown in Fig. 3, at one end of the rod, loop holder 11 is formed with sufficient height to securely retain one of the loops 14 on stent holding wire 10. Similarly, at the other end of stent holding frame 13, the other loop holder 12 also is formed from the rod with sufficient height to retain the second loop 14 on stent holding wire 10. Further, as illustrated in Fig. 3, the height of loop holder 12 may be extended a sufficient distance to form a handle 16.

Fig. 5 illustrates the arrangement of stent holding wire 10 at an end of a stent held in the holder. Stent holding wire 10 passes through the center of stent 9. The size of loop 14 and the length of stent holding wire 10 have been selected to ensure that the welded joint at position 15 remains far enough within the center of stent 9, and that the sides of loop 14 are spread by its loop holder (not shown) far enough to ensure the loop sides contact the inside edge of the end of stent 9 at diametrically-opposed positions 17.

The stent is mounted on the stent holder by first passing one of the loops 14 of stent holding wire 10 through the center of stent 9 until a portion of both loops 14 protrude from their respective ends of stent 9, and then one loop 14 is passed over a loop holder 11 or 12. Alternatively, one of the loops 14 may be first passed over a loop holder, then the other loop 14 may be passed through the center of stent 9. If handle 16 has been formed in stent holding frame 13, the first loop should be passed over loop holder 12. The remaining loop holder 11 is then gently pushed toward loop holder 12 far enough to permit the remaining free loop 14 to be passed over loop holder 11. When loop holder 11 is then released, the stent holding frame 13 acts as a spring to apply tension to the ends of stent holding wire 10 through loop holders 11 and 12, and the forces applied by the sides of loops 14 to their respective ends of stent 9 securely locate the stent both axially and transversely between loop holders 11 and 12.

The foregoing example provides a stent coating holder which: securely holds a stent without the need to exert large compressive or tensile forces, thereby minimizing the risk of stent deformation or misalignment due to application of excessive retaining or positioning forces ; is relatively inexpensive to produce ; may be readily adapted to be used with a range of stent lengths and diameters; minimizes coating spray shadows; and eliminates the need for any of the frequently-replaced wire securement bushings used in the prior art. This example is also amenable to use in automatic loading machines for automation of the stent loading and coating process.

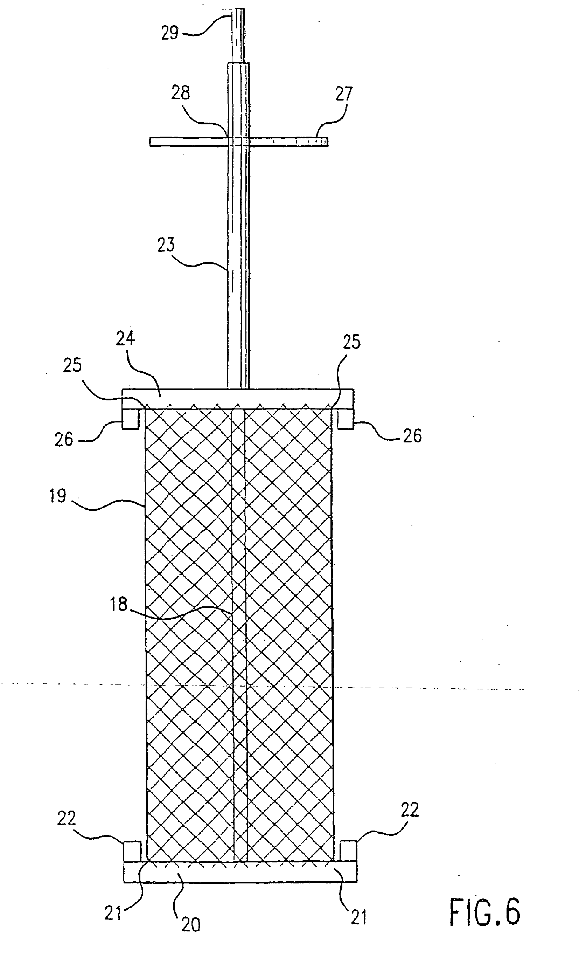

Fig. 6 illustrates a second example. In this example, a center rod 18 passes through the center of a stent 19. At one end of stent 19 is a lower crossbar 20 affixed to center rod 18. One end of stent 19 rests upon lower crossbar 20 and contacts the crossbar at two diametrically opposed contact points 21. If, as shown in this example, the contact points 21 are recessed from the end of stent 19, lower crossbar 20 will engage the end of stent 19 in a manner that ensures that stent 19 rotates at the same angular velocity as lower crossbar 20 during high speed rotation of the stent in the spin- dipping coating application process. Lower crossbar 20 has two small projections 22 at each end of the crossbar, facing stent 19. The crossbar projections 22 preclude radial movement and resulting misalignment of stent 19 during high speed rotation of the stent in the spin- dipping coating application process.

After stent 19 is placed over center rod 18, a center rod guide tube 23 with an upper crossbar 24 affixed thereto slips over center rod 18 and slides toward lower crossbar 20 until upper crossbar 24 contacts the remaining free end of stent 19 at two diametrically opposed contact points 25. Center rod18 extends beyond a spinning machine end 29 of center rod guide tube 23 and is driven to rotate about its longitudinal axis by a spinning machine (not shown). As with lower crossbar 20, if the two contact points 25 are recessed from the second end of stent 19, upper crossbar 24 will engage the second end of stent 19 in a manner that ensures that stent 19 rotates at the same angular velocity as upper crossbar 20 during high speed rotation. Upper crossbar 24 also has two small projections 26 at each end of the crossbar, facing stent 19. The crossbar projections 26, like those on lower crossbar 20, also serve to preclude radial movement and resulting misalignment of stent 19 during high speed rotation of the stent.

Once upper crossbar 24 comes into contact with stent 19, a locking pin 27 is inserted through transverse position locking holes28 in center rod 18 and center rod guide tube 23 (center rod locking hole not shown) to fix the orientation of center rod guide tube 23 relative to center rod 18 in both the axial and rotational directions. Such fixation by locking pin 27 applies a light axial compressive force through contact points 21 and 25 onto stent 19, and ensures that upper crossbar 24 may not move farther away from lower crossbar 20 during the spin-dipping coating application process, thereby permitting stent 19 to become misaligned within the stent coating holder. Locking pin 27 further precludes rotation of upper crossbar 24 relative to lower crossbar 20, which again could result in stent misalignment.

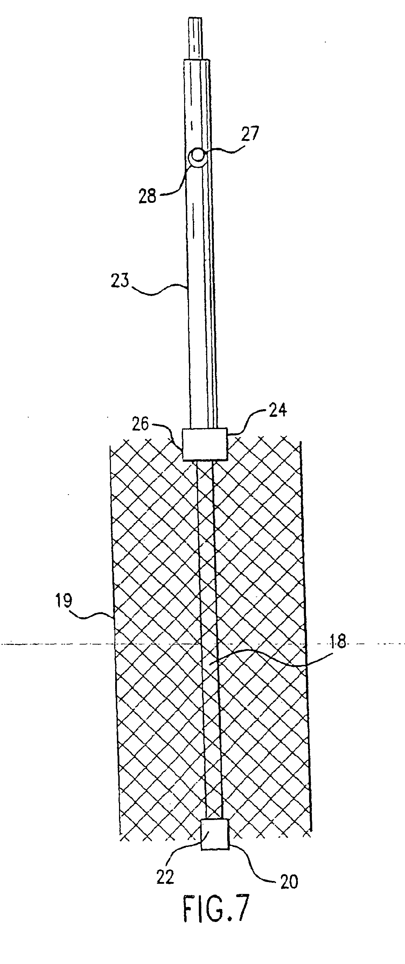

Fig. 7 is a side view of the stent coating holder of the second example illustrated in Fig. 6, viewed from a position rotated 90 degrees about the longitudinal axis of the stent coating holder from the viewpoint in Fig. 6. As shown in Fig. 7, lower crossbar 20, upper cross bar 24, locking pin 27, center rod 18 and center rod guide tube 23 are generally located in the same plane, with lower crossbar 20, upper cross bar 24 and locking pin 27 approximately parallel to each other and perpendicular to center rod 18 and center rod guide tube 23. This configuration aids in co-linear application of the light compressive force on the ends of stent 19 between lower crossbar 20 and upper crossbar 24. The present invention is not, however, limited to this configuration, as none of lower crossbar 20, upper cross bar 24 and locking pin 27 need lie in the same plane as the other two as long as the non-co-planar light compressive forces applied to the stent by lower crossbar 20 and upper crossbar 22 do not distort the stent. Further, in order to facilitate use of this stent coating holder with stents of different lengths, a number of locking pin holes may be located along the length of either or both center rod 18 and center rod guide tube 23.

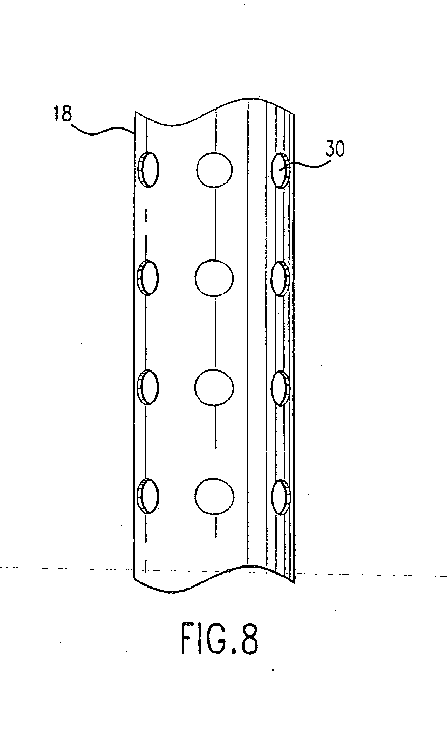

Fig.8 is a side view of a portion of a modified center rod 18 of the second example of the stent coating holder of the present invention. In this modification, center rod 18 is a tube with a plurality of holes 30 therein along the portion of the center rod within the center of stent 19. The holes 30 permit air to be introduced into the end of center rod 18 held by the spinning machine (not shown) and thence directed out of holes 30 onto the inner surface of stent 19. The air directed onto the stent in this manner will facilitate drying of the coating applied to the stent during the spin-dipping process, helping to ensure homogeneous distribution of the coating on stent 19, minimal covering of the gaps or "windows" between the elements of the stent by films of the coating material, and minimizing the time required to dry the coated stent to permit increased production rates.

The foregoing second example shares many of the advantages of the first example, in that it provides a stent coating holder which securely holds a stent without the need to exert large compressive or tensile forces and thereby minimizes the risk of stent deformation or misalignment due to application of excessive retaining or positioning forces; it is relatively inexpensive to produce, and it may be readily adapted to be used with a variety of stent lengths and diameters. The second example has the additional advantages of providing a simple approach to introduction of drying air within the center of the stent following coating, and providing a stent coating holder that does not require any holder apparatus extending beyond the lower stent retaining crossbar, thus minimizing space and volume requirements of the spin-dipping stent coating equipment and related containers with which this example is to be used.

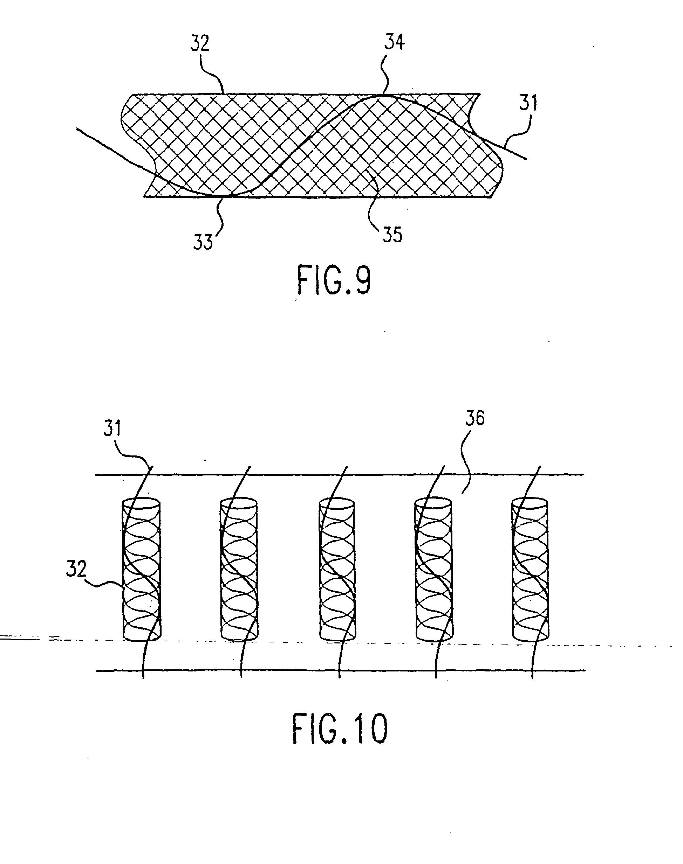

Fig. 9 illustrates a third example. In this example, stent holding wire 31 is shown in cross-section view passing through the center of stent 32. Stent holding wire 31 has two bends formed prior to insertion through stent 32 which contact the inner surface of stent 32 at two locations 33,34 and hold stent 32 for coating deposition, for example by spraying with, or immersion into, a coating material. The wire may be composed of any material, such as stainless steel, with sufficient tensile and bending strength to withstand the loads imposed on the wire during stent loading, holding and unloading, while also being small enough in diameter to minimize coating shadowing. Preferably, the diameter of the stent holding wire is less than the thickness of the struts 35 comprising stent 32, and the wire is amendable to having the bends formed by cold working.

In this example, stent 32 may be mounted on stent holding wire 31 by placing one end of stent holding wire 31 through one end stent 32 sufficiently far to permit the wire to be grasped and drawn out the other end of stent 32. Sufficient tension may then be applied to the ends of the wire to elastically straighten the bends 33,34 until the inner diameter of stent 32 will pass over flattened bends 33,34. Once stent 32 is located over both stent holding bends 33,34, the tension on wire 31 may be relaxed, permitting bends 33,34 to elastically contract toward their originalun-tensioned states until they contact the inner diameter of stent 32. Following application of the stent coating, stent 32 may be removed by reversing the foregoing process, i. e. , by applying tension to the ends of stent holding wire 31 until bends 33,34 contract sufficiently for coated stent 32 to be moved off the bent portions of wire31, and then releasing tension so that the end of stent holding wire 31 adjacent to stent 32 may be released from the grasp of the tensioning device and backed out through the center of stent 32.

The stent holding wire in this third example is well suited to use in automated stent coating processes. For example, as schematically shown in Fig. 10, once stent holding wire 31 is positioned within stent 32 (an operation that, for instance, may have been performed in the stent manufacturer's plant), the assembled stents and holders may be affixed to a continuous carrier, such as a carrier tape 36 shown in Fig. 10, and wound onto a reel (not shown). Alternatively, the ends of the stent holding wires could be affixed between two continuous parallel wires in a configuration similar to carrier tape 36 and similarly rolled onto a reel. The loaded reel may then be transported to a stent coating facility for feeding into a stent coating apparatus. Such an apparatus could then unwind the continuous stent- bearing carrier tape, exposing the stents to sequential removal from the carrier by a robotic "pick-and-place"apparatus which grasps the ends of the stent holding wires for transport to the coating deposition portion of the stent coating apparatus. The same robotic pick-and- place equipment could then transport the coated stents to other areas for performance of other operations, such as inspection, and then apply tension to the stent holding wires to unload the coated stents. An automated stent coating process such as the foregoing would permit high speed, consistent, repeatable, accurate and low cost stent loading, handling, coating and unloading.

It should be understood that the foregoing description of the third example alternative carrier is not intended to be limiting, an a number of modifications and alternatives may be employed consistent with this example. For example, in addition to using conventional materials for stent holding wire 31 such as stainless steel, materials may be used that permit the expansion and contraction of bends in the wire by other than application or removal of axial tension. Nitinol is such an alternative material. When the temperature of a Nitinol wire rises, the wire will bend, and when cooled will straighten. Accordingly, a cool, substantially straight Nitinol wire could be inserted through the center of a stent and then heated (by means such as conduction, convention, radiation or electrical resistance heating) until it contacted the inner surface of the stent. After coating deposition, the Nitinol wire could then be straightened by cooling for coated stent unloading. Consistent bending patterns could be obtained by employing Nitinol stent holders supplied with pre-formed bends or "kinks" which would serve as bend initiation points.

Other alternatives within the scope of the third example include use of additional holding wire bends within the inner diameter of the stent to increase the number of stent contact points and thereby improve the holding wire's grip on the stent. Improved grip and stent stability could also be obtained by pre-forming the stent holding wire bends in a manner that permits more than two bends to contact the inner surface of the stent at additional radial locations beyond the 180 degree-separation of a two-bend holding wire.

As with the first and second examples, the third example provides a stent coating holder which: securely holds a stent without the need to exert large compressive or tensile forces, thereby minimizing the risk of stent deformation or misalignment due to application of excessive retaining or positioning forces; is simple and relatively inexpensive to manufacture and use (depending on the materials and configuration selected, inexpensive enough to permit one-time, disposable use and thus further simplifying the stent coating production process); is readily amenable to high-volume, high consistent stent coating operations; is virtually free of coating shadowing problems; and may be readily adapted to be used with a variety of stent lengths and diameters.

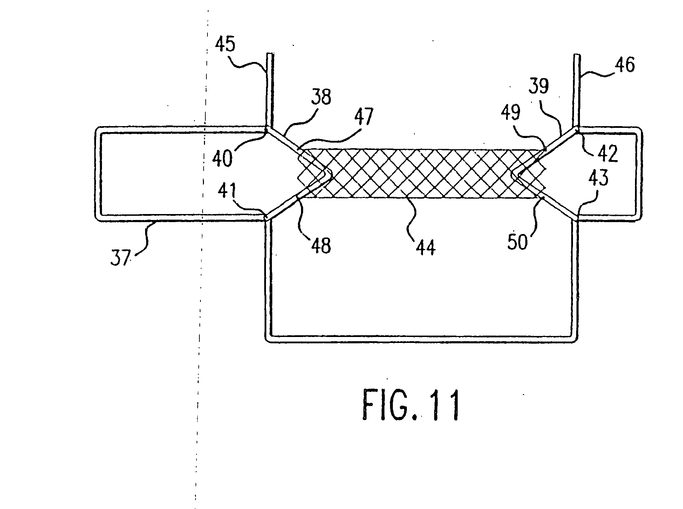

An embodiment of the present invention is illustrated in Fig. 11. In this embodiment, a holder frame 37 is provided. As in the first example, the frame may be formed from a 1.0 mm diameter stainless steel rod press-formed into the desired shape. Frame 37 alternatively may be economically formed from a molded plastic. Affixed to frame 37 are two "V"-shaped stent holding points 38 and 39. Stent holding points38 and39may be formed from metal wire, for example 101.6 µm [0.004 inch] diameter wire, or molded plastic, and are affixed to the frame at locations 40, 41 and 42, 43, respectively, by, for example, welding or use of a suitable adhesive. Alternatively, stent holding points 38 and 39 may be integrally formed with frame 37, for example, by suitably bending the frame wire or by including the stent holding points in the mold of a plastic frame.

Stent 44 is mounted onto the stent holder of the embodiment by elastically spreading frame 37 apart at points 45 and 46 a distance sufficient to permit stent 44 to be placed between stent holding points 38 and 39, and then holding stent 44 between stent holding points 38 and 39 while frame 37 is released, thereby permitting the tips of stent holding points 38 and 39 to enter the ends of stent 44 until the stent is contacted at interior end surfaces 47, 48 and 49,50, respectively. Stent 44 is held firmly between stent holding points 38 and 39 by light compressive forces applied by frame 37, which is sized to ensure a compressive load is applied to the ends of stent 44 when frame 37 is permitted to elastically return toward its unloaded position. The stent holder is unloaded by simply spreading the frame again to disengage the tips of stent holding points 38 and 39 from the ends of stent 44. This embodiment of the present invention, like the foregoing example, provides a stent holder that is inexpensive and simple to manufacture, minimizes stent coating shadows, and is very easy to rapidly and reliably load and unload during a stent coating production process.

In addition to the foregoing examples, the present invention encompasses methods of use of above stent holders. The method of use of the first example comprises the steps of passing an end of a center wire of desired length through the center of a stent to which a coating is to be applied, placing a loop of a desired size formed at each end of the center wire over loop holders on opposing ends of a stent coating holder such that tension is applied to the center wire and the loop holders cause the sides of each loop to contact an end edge of the loops'respective ends of the stent, and applying a desired coating to the stent loaded on the stent coating holder. There is no required order for the steps of passing the wire through the stent and placing a first loop over a loop holder on the stent coating holder.

A second method of use comprises passing a center rod through a stent until a first end of the stent contacts a lower crossbar affixed to the center rod, then sliding the end of the center rod opposite the lower crossbar into a center rod guide tube until an upper crossbar affixed to the center rod guide tube contacts the second end of the stent, inserting a locking pin into corresponding transverse holes through the center rod and center rod guide tube to fix the stent between the lower and upper crossbars, placing the assembled stent coating holder into a stent coating applicator and applying a desired coating to the stent.

A third method of use comprises passing one end of a stent holding wire through the center of a stent, causing the holding wire to elongate and reduce in transverse width until the stent is positioned over bends in the holding wire, causing the stent holding wire to contract and expand laterally until the holding wire contacts the inner surface of the stent at least points, depositing a coating on the stent, then causing the holding wire to again elongate and reduce in transverse width until the holding wire can be removed from the center of the coated stent.

A fourth method of use of the stent holder according the present invention comprises elastically spreading a stent holder frame equipped with opposing stent holding points, inserting the stent between the stent holding points, and releasing the frame to permit the stent holding points to enter the ends of the stent until they rest against opposing points on the interior diameter of the stent's ends. Following coating, the stent frame is again elastically spread, and the stent is removed from the stent holding points.

While the present invention has been described with reference to what is presently considered to be preferred embodiment thereof, it is to be understood that the present invention is not limited to the disclosed embodiment or construction. On the contrary, the present invention is intended to cover various modifications and equivalent arrangements within the scope of appended claims.

## Claims

1. A stent holder for holding a stent (44) during application of a stent coating,
**characterised in that** it comprises:
a frame (37), including a first frame side (45) opposite a second frame side (46), wherein the first frame side (45) and second frame side (46) are elastically spreadable from one another;
at least one stent holding projection (38) located on the first frame side;
and at least one stent holding projection (39) located on the second frame side (46), wherein the first frame side (45) and the second frame side (46) are separated by a distance such that when the stent (44) is placed between at least one stent holding projection (38) on the first frame side (45) and at least one stent holding projection (39) on the second frame side (46), the stent (44) is held in axial compression by the stent holding projections (38; 39).

2. The stent holder of claim 1, wherein the stent holding projections (38; 39) are v-shaped.

3. The stent holder of claim 2, wherein the frame (37) is formed from a metal wire.

4. The stent holder of claim 3, wherein the metal wire has a nominal diameter of between 0.5 mm and 5 mm.

5. The stent holder of claim 4, wherein the stent holding projections (38; 39) are formed from a metal wire.

6. The stent holder of claim 1, wherein the metal wire forming the stent holding projections (38; 39) has a nominal diameter of between 50.8 µm and 254 µm.

7. A method for using the stent coating holder of one of the preceding claims, comprising the steps of:
elastically spreading the first frame side (45) away from the second frame side (46) until the opposing stent holding projections (38; 39) are sufficiently separated to permit the stent to be placed between the opposing stent holding projections (38; 39);
placing the stent (44) between the opposing stent holding projections (38; 39);
permitting the opposing stent holding projections (38; 39) to enter the ends (47, 48, 49, 50) of the stent (44) and apply an axial compressive force to the ends (47, 48, 49, 50) of the stent (44); and
applying the stent coating to the stent (44).

## Patentansprüche

1. Stenthalterung zum Halten eines Stents (44) während der Aufbringung einer Stentbeschichtung, **dadurch gekennzeichnet, dass** sie umfasst:
- einen Rahmen (37), welcher eine erste Rahmenseite (45) gegenüber einer zweiten Rahmenseite (46) einschließt, wobei die erste Rahmenseite (45) und die zweite Rahmenseite (46) voneinander elastisch abspreizbar sind;
- zumindest einen Stenthalterungsvorsprung (38), welcher sich auf der ersten Rahmenseite befindet;
- zumindest einen Stenthalterungsvorsprung (39), welcher sich auf der zweiten Rahmenseite (46) befindet, wobei die erste Rahmenseite (45) und die zweite Rahmenseite (46) durch einen Abstand voneinander getrennt sind, so dass, wenn der Stent (44) zwischen den zumindest einen Stenthalterungsvorsprung (38) auf der ersten Rahmenseite (45) und den zumindest einen Stenthalterungsvorsprung (39) auf der zweiten Rahmenseite (46) angeordnet ist, der Stent (44) in axialem Druck durch die Stenthalterungsvorsprünge (38; 39) gehalten ist.

2. Stenthalterung nach Anspruch 1, wobei die Stenthalterungsvorsprünge (38; 39) V-förmig sind.

3. Stenthalterung nach Anspruch 2, wobei der Rahmen (37) aus einem Metalldraht gebildet ist.

4. Stenthalterung nach Anspruch 3, wobei der Metalldraht einen nominalen Durchmesser zwischen 0,5 mm und 5 mm aufweist.

5. Stenthalterung nach Anspruch 4, wobei die Stenthalterungsvorsprünge (38; 39) aus einem Metalldraht gebildet sind.

6. Stenthalterung nach Anspruch 1, wobei der Metalldraht, welcher die Stenthalterungsvorsprünge (38; 39) bildet, einen nominalen Durchmesser zwischen 50.8 µm und 254 µm aufweist.

7. Verfahren zur Verwendung der Stentbeschichtungshalterung nach einem der vorherigen Ansprüche, umfassend die Schritte:
- elastisches Spreizen der ersten Rahmenseite (45) weg von der zweiten Rahmenseite (46) bis die gegenüberliegenden Stenthalterungsvorsprünge (38; 39) ausreichend getrennt sind, um das Platzieren des Stents zwischen den gegenüberliegenden Stenthalterungsvorsprüngen (38; 39) zu erlauben;
- Positionieren des Stents (44) zwischen den gegenüberliegenden Stenthalterungsvorsprünge (38; 39);
- Erlauben, dass die gegenüberliegenden Stenthalterungsvorsprünge (38; 39) in die Enden (47, 48, 49, 50) des Stents (44) eintreten, und eine axiale Druckkraft auf die Enden (47, 48, 49, 50) des Stents (44) aufbringen; und
- Anwenden der Stentbeschichtung auf den Stent (44).

## Revendications

1. Un support de stent pour supporter un stent (44) lors de l'application d'un revêtement de stent, **caractérisé en ce qu'**il comprend :
- un cadre (37) comprenant un premier côté de cadre (45) en vis-à-vis d'un second côté de cadre (46), où le première premier côté de cadre (45) et le second côté de cadre (46) sont élastiquement déployables l'un par rapport à l'autre ;
- au moins une saillie support de stent (38) située sur le premier côté du cadre ;
- et au moins une saillie support de stent (39) située sur le second côté de cadre (46), où le premier côté de cadre (45) et le second côté de cadre (46) sont séparés d'une distance telle que lorsque le stent est placé entre au moins une saillie support de stent (38) sur le premier côté de cadre (45) et au moins une saillie support de stent (39) sur le second côté de cadre (46), le stent (44) soit maintenu en compression axiale par les saillies supports de stent (38 ; 39).

2. Le support de stent de la revendication 1, où les saillies supports de stent (38 ; 39) sont en forme de v.

3. Le support de stent de la revendication 2, où le cadre (37) est formé à partir d'un fil métallique.

4. Le support de stent de la revendication 3, où le fil métallique présente un diamètre nominal compris entre 0,5 mm et 5 mm.

5. Le support de stent de la revendication 4, où les saillies supports de stent (38 ; 39) sont formées à partir d'un fil métallique.

6. Le support de stent de la revendication 1, où le fil métallique formant les saillies supports de stent (38 ; 39) présente un diamètre nominal compris entre 50,8 µm et 254 µm.

7. Un procédé d'utilisation du support de revétement de stent de l'une des revendications précédentes, comprenant les étapes consistant à :
- déployer élastiquement le premier côté de cadre (45) en éloignement du second côté de cadre (46) jusqu'à ce que les saillies supports de stent en vis-à-vis (38 ; 39) soient suffisamment séparées pour permettre de placer le stent entre les saillies supports de stent en vis-à-vis (38 ; 39) ;
- placer le stent (44) entre les saillies supports de stent en vis-à-vis (38 ; 39) ;
- permettre aux saillies supports de stent en vis-à-vis (38 ; 39) d'entrer dans les extrémités (47, 48, 49, 50) du stent (44) et appliquer une force de compression axiale aux extrémités (47, 48, 49, 50) du stent (44) ; et
- appliquer le revêtement de stent sur le stent (44).
